# EUROPEAN PATENT APPLICATION

(11) **EP 4 749 045 A1**
(43) Date of publication of application: **27.05.2026**
(21) Application number: 24214865.8
(22) Date of filing: 22.11.2024
(51) Int. Cl.: E04B 1/00, A61L 2/22

(54) **DECONTAMINATION APPARATUS AND METHOD FOR RENDERING MICROBIAL FOULING INNOCUOUS**

(71) Applicant: Reddo Floor Solutions AB, 102 47 Stockholm (SE)
(72) Inventor: ÅHSBERG, Johan, 474 70 Mollösund (SE)
(74) Representative: Brann AB

(57) **Abstract**

The present invention relates to a decontamination apparatus (1) for rendering microbial fouling between a flooring (3) and a subfloor (4) innocuous without removing the flooring (3). The decontamination apparatus (1) comprises a main body (6) configured to be connected to the flooring (3) at an opening (7) in the flooring (3), an elevating device (12) that is connected to the main body (6) and is displaceable in the vertical direction between an extended position and a retracted position in relation to the main body (6), wherein the elevating device (12) is configured to engage the subfloor (4) when located in the extended position, in order to elevate a portion of the flooring (3) from the subfloor (4) and create a space (5) therebetween, and a nozzle device (16) that comprises at least one nozzle (17), wherein the nozzle device (16) is displaceable in the vertical direction between an extended position and a retracted position in relation to the main body (6), wherein said at least one nozzle (17) is configured to be located in said space (5) between the flooring (3) and the subfloor (4) when the nozzle device (16) is located in the extended position, wherein said at least one nozzle (17) of the nozzle device (16) is configured to be connected to a source (20) of pressurized decontamination liquid (19).

## Description

### Technical field of the invention

The present invention relates in general to the field of method and apparatus for drying water damaged floor having a flooring and a subfloor. The subfloor is a support layer and may for instance be constituted by concrete and/or wooden particle-boards, etc., and the flooring is the top layer of the floor and may for instance be constituted by floating flooring made of parquet and/or wood planks, etc. Such floor may also comprise a subsurface layer located between the flooring and the subfloor.

The present invention relates specifically to a decontamination method and decontamination apparatus for rendering microbial fouling between a flooring and a subfloor innocuous without removing the flooring.

### Background of the invention

Unfortunately, it is quite common that floors in houses and flats become water damaged, usually due to leaking pipes, refrigerators, freezers, dishwashers, washing machines, aquariums, etc. The owner as well as the insurance company wish that the flooring does not need to be replaced, instead they aim at trying to dry the water damaged floor without exchanging the flooring in order to save cost. There are numerous known methods and apparatuses configured for drying water damaged floor by blowing dry/hot air into a space generated between the flooring and the subfloor, and/or by sucking moist air from the space between the flooring and the subfloor. For instance EP3268549, SE543309, SE544844 disclose different successful methods and apparatus/equipment for drying water damaged floor having a flooring and a subfloor.

However, in situations where microbial fouling is present, but not detected before the drying process is performed, known drying methods and apparatuses may increase the risk of spreading bacteria, spores, and bad smelling to the adjoining structures and to the ambient environment.

Situations where microbial fouling is detected between the flooring and the subfloor, the microbial fouling usually grows when there is a small and/or intermittent leakage from a machine or from water/sewer pipes. Thus, the water damage might not be visible or noticeable and may occur for a long period of time, i.e. weeks or months, before detected. Other situations are for instance water damage in a summer-vacation house, wherein the actual water damage happened during the winter period. In such situations it is common that microbial fouling, e.g. spores and bacteria, has started to spread in the space between the flooring and the subfloor. For different reasons the only decontamination strategy used today is to remove the entire flooring and sometimes also the subfloor, in order to get rid of the microbial fouling. However, removing the flooring having microbial fouling will actually increase the spreading of bacteria, spores, and bad smelling to the adjoining structures and the ambient environment.

There is a need in the field for a reliable, cheap and unharmful method and apparatus for decontaminating microbial fouling that is present between a flooring and a subfloor due to water damage and/or humid climate.

### Object of the invention

The present invention aims at obviating the aforementioned and other disadvantages and failings of previously known methods and apparatus for decontaminating a water damaged floor having microbial fouling, and at providing an improved method and apparatus for rendering microbial fouling between a flooring and a subfloor innocuous. Thus, the aim is to decontaminate the floor in an efficient and quick way without having to remove or replace the flooring.

A primary object of the present invention is to provide an improved method and apparatus configured for rendering microbial fouling between a flooring and a subfloor innocuous without removing the flooring. It is another object of the present invention to provide an improved method and apparatus for rendering microbial fouling between a flooring and a subfloor innocuous, wherein the process is cheap and reliable. It is another object of the present invention to provide an improved method and apparatus for rendering microbial fouling between a flooring and a subfloor innocuous, wherein the process is unharmful. It is another object of the present invention to provide an improved method and apparatus for rendering microbial fouling between a flooring and a subfloor innocuous, wherein the flooring/subfloor does not need to be replaced after a successful drying operation.

### Summary of the invention

According to the invention at least the primary object is attained by means of the initially defined method and decontamination apparatus having the features defined in the independent claims. Preferred embodiments of the present invention are further defined in the dependent claims.

According to a first aspect of the present invention, there is provided a decontamination apparatus of the initially defined type, wherein decontamination apparatus comprises:
- a main body configured to be connected to the flooring at an opening in the flooring,
- an elevating device that is connected to the main body and is displaceable in the vertical direction between an extended position and a retracted position in relation to the main body, wherein the elevating device is configured to engage the subfloor when located in the extended position, in order to elevate a portion of the flooring from the subfloor and create a space therebetween, and
- a nozzle device that comprises at least one nozzle, wherein the nozzle device is displaceable in the vertical direction between an extended position and a retracted position in relation to the main body, wherein said at least one nozzle is configured to be located in said space between the flooring and the subfloor when the nozzle device is located in the extended position,
wherein said at least one nozzle of the nozzle device is configured to be connected to a source of pressurized decontamination liquid.

According to a second aspect of the present invention, there is provided a decontamination method of the initially defined type, wherein the method use said decontamination apparatus and comprises the steps of:
- forming an opening in the flooring in connection with a portion of the flooring subject to microbial fouling,
- connecting a main body of the decontamination apparatus to the flooring at said opening,
- elevating a portion of the flooring from the subfloor and creating a space therebetween by means of an elevating device of the decontamination apparatus, and
- applying pressurized decontamination liquid into the space between the flooring and the subfloor by means of a nozzle device of the decontamination apparatus, wherein the nozzle device comprises at least one nozzle located in said space during application of the pressurized decontamination liquid.

Thus, the present invention is based on the insight/knowledge that microbial fouling that has been made innocuous does not need to be removed and the affected flooring does not need to be removed either. Instead, the water damaged flooring may be dried in a conventional way after the inventive decontamination process without running the risk of spreading the spores, bacteria and bad smelling to the adjoining structures and to the ambient environment. The present invention is based on the idea to distribute the decontamination liquid directly onto the affected surfaces, without the need to remove the flooring. The decontamination liquid has essentially instant effect.

The present invention provides the advantage that the flooring does not need to be replaced due to the microbial fouling, and the process is cheap, reliable and unharmful.

According to various embodiments of the present invention, the elevating device and the nozzle device are connected to each other and jointly displaceable in relation to the main body. Thereby the manipulation of the decontamination apparatus is made easier and the risk of erroneously damaging the nozzle device, when lowering the flooring after the application of the pressurized decontamination liquid, is avoided.

According to various embodiments of the present invention, the nozzle device comprises at least three nozzles, preferably at least six nozzles. Thereby the distribution of the decontamination liquid is more efficient.

According to various embodiments of the present invention, the source of pressurized decontamination liquid is constituted by a pressure tank, and wherein a conduit extends between the nozzle device and said tank, wherein the conduit comprises a controllable valve. Thereby the pressure in the pressure tank may be controlled/adjusted before activation in order to obtain optimal distribution of the decontamination liquid.

According to various embodiments of the present invention, the method after the step of applying pressurized decontamination liquid, further comprises the step of:
- lowering the portion of the flooring into contact with the subfloor after the application of the pressurized decontamination liquid, in order to further spread out the applied decontamination liquid.

Further advantages with and features of the invention will be apparent from the following detailed description of preferred embodiments.

### Brief description of the drawings

A more complete understanding of the abovementioned and other features and advantages of the present invention will be apparent from the following detailed description of preferred embodiments in conjunction with the appended drawings, wherein:
- Fig. 1: is a schematic cross-sectional side view of a decontamination apparatus according to a first embodiment, wherein the elevating device and the nozzle device are in the retracted positions,
- Fig. 2: is a schematic illustration of a decontamination apparatus according to a first embodiment, wherein the elevating device and the nozzle device are in the extended positions,
- Fig. 3: is a schematic cross-sectional side view of a decontamination apparatus according to a second embodiment, wherein the decontamination apparatus is connected to the flooring and the flooring is not elevated,
- Fig. 4: is a schematic illustration of the decontamination apparatus according to figure 3, wherein the flooring is elevated,
- Fig. 5: is a schematic cross-sectional side view of a decontamination apparatus according to a third embodiment, wherein the elevating device and the nozzle device are in the retracted positions,
- Fig. 6: is a schematic perspective view from above of an attachment ring,
- Fig. 7: is a schematic cross-sectional side view of a decontamination apparatus according to a fourth embodiment, wherein the elevating device and the nozzle device are in the retracted positions, and
- Fig. 8: is a schematic view from above of a nozzle device.

### Detailed description of preferred embodiments of the invention

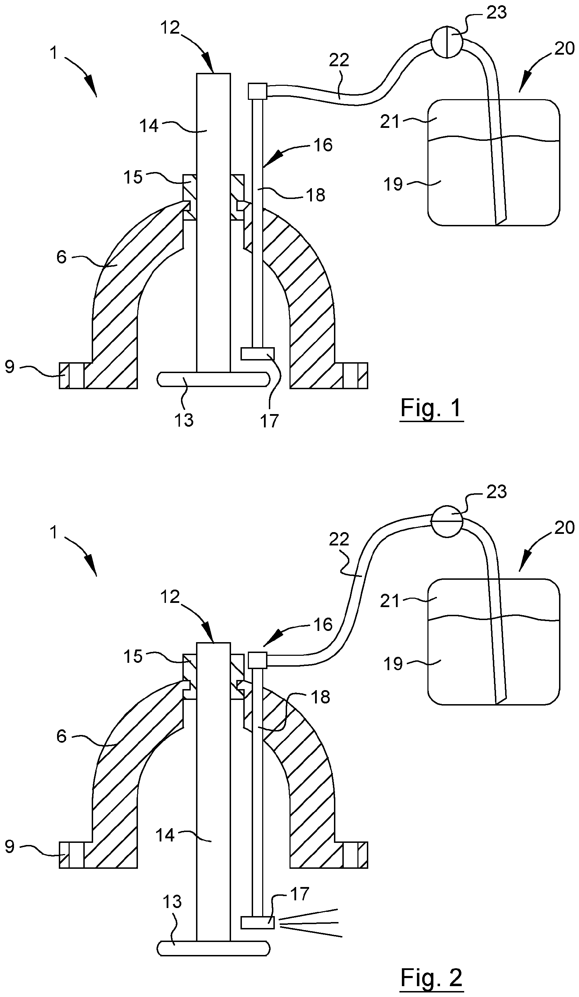

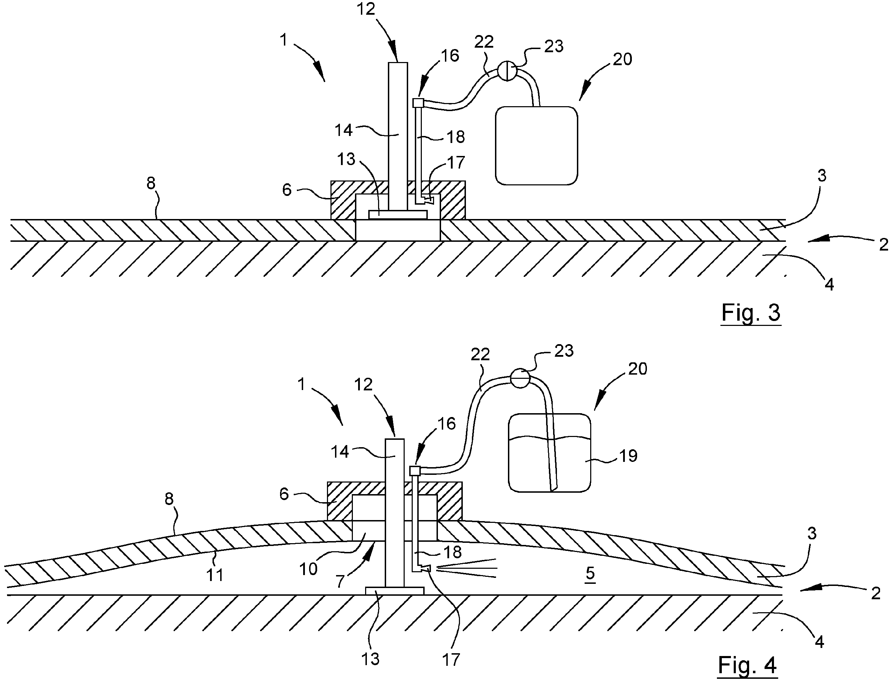

The present invention relates in general to the field of repairing a water damage without having to remove/exchange the flooring. Reference is initially made to figures 1-4, disclosing a schematic illustration of an inventive decontamination apparatus, generally designated 1, and the inventive method. Figures 1 and 2 disclose a decontamination apparatus 1 according to a first embodiment, and figures 3 and 4 disclose a decontamination apparatus 1 according to a second embodiment.

The inventive decontamination apparatus 1 is intended to be used to decontaminate a floor 2 having a flooring 3 and a subfloor 4, wherein microbial fouling has appeared and contaminated an area in the space 5 between the flooring 3 and the subfloor 4. Thus, the microbial fouling may be present at the flooring 3 and/or at the subfloor 4. The subfloor 4 is a support layer may for instance be constituted by concrete and/or wooden particle-boards, etc. The flooring 3 is the top layer of the floor 2 and is a so-called floating flooring and may for instance be constituted by a wooden flooring such as parquet and/or wood planks, etc. The floor 2 may also comprise a subsurface layer located in the space 5 between the flooring 3 and the subfloor 4, wherein the subsurface layer a resilient and/or noise cancelling layer that may be constituted by a synthetic foam, lump cardboard, etc. The subsurface layer may also be constituted by a vapor barrier. Thus, it shall be pointed out that the subsurface layer may be water/vapor permeable or water/vapor non-permeable.

The decontamination apparatus 1 comprises a main body or housing 6 that is configured to be releasably connected the flooring 3. Before the decontamination apparatus 1 is connected to the flooring 3, an opening or hole 7 is provided in the flooring 3. The opening 7 may have any suitable shape, such as circular, rectangular, polygonal, etc. The opening 7 may be located at the edge of or adjacent the contaminated area, or may be located overlapping the contaminated area. The opening 7 is configured to provide access to the space 5 between the flooring 3 and the subfloor 4 from above the flooring 3. The subsurface layer may also be provided with an opening overlapping the opening 7 in the flooring 3 in order to secure access to the portion of the space 5 located below the subsurface layer as well as to the portion of the space 5 located above the subsurface layer.

The main body 6 of the decontamination apparatus 1 is configured to be connected to the flooring 3 at said opening 7. According to one example, the main body 6 is connected to the top surface 8 of the flooring 3, for instance by means of a plurality of screws extending through a flange 9 of the main body 6. According to another example, the main body 6 is connected to the flooring 3 by engaging the inner wall 10 of the opening 7, for instance by means of spring-loaded pins. According to another example, the main body 6 is connected to the flooring 3 by engaging the lower surface 11 of the flooring 3 adjacent the opening 7, for instance by hooks extended through the opening 7 and into the space 5.

The decontamination apparatus 1 further comprises an elevating device, generally designated 12, wherein the elevating device 12 is connected to the main body 6. The elevating device 12 is displaceable in the vertical direction between a retracted position and an extended position in relation to the main body 6. In figures 1 and 3 the elevating device 12 is in the retracted position, and in figures 2 and 4 the elevating device 12 is in the extended position. When the main body 6 is being attached to the flooring 3, the elevating device 12 shall be in the retracted position in order to not obstruct proper connection of the decontamination apparatus 1 to the flooring 3. Thus, when the elevating device 12 is in the retracted position, it does not engage the subfloor 4. When the main body 6 is connected to the flooring 3 and the elevating device 12 is in the extended position, the elevating device 12 extend through the opening 7 in the flooring 3.

The elevating device 12 is configured to engage the subfloor 4 when displaced from the retracted position to the extended position, and at the same time a portion of the flooring 3 surrounding the opening 7 is elevated from the subfloor 4, wherein a space 5 is created between the flooring 3 and the subfloor 4. The elevating device 12 may engage the subfloor 4 directly, or indirectly via the subsurface layer.

According to various embodiments, the elevating device 12 comprises a foot 13 and a stem 14, wherein the foot 13 is connected to the lower end of the stem 14 and the stem 14 extends in the vertical direction. According to various embodiments, the foot 13 is rotatably connected to the stem 14. According to various embodiments, the stem 14 is provided with an external thread and the housing 6 is provided with a corresponding internal thread, wherein the elevating device 12 is displaced by turning the stem 14 in relation to the main body 6. The upper end of the stem 14 may be equipped with a wing nut or lever arm for facilitating turning of the stem 14. Alternatively or in addition, the upper end of the stem 14 may have a shape configured to be engaged with a suitable tool, such as a wrench or an Allen key. The foot 13 is configured to increase the contact area between the elevating device 12 and the subfloor 4, and is configured to make it easier to turn the stem 14. According to alternative embodiments, the stem 14 is rotated by means of an electric motor. When the elevating device 12 is displaced from the extended position to the retracted position, the flooring 3 will return to its initial shape and once more rest against the subfloor 4.

According to various embodiments, for instance as disclosed in figures 1 and 2, the main body 6 comprises a sleeve 15 located at the interface between the stem 14 and the main body 6. The sleeve 15 is rotatably arranged in the main body 6 and is provided with an internal thread that cooperates with the external thread of the stem 14. By turning the sleeve 15, the elevating device 12 will be displaced in the vertical direction without turning in relation to the main body 6. The foot 13 is preferably provided with engagement means at the lower surface in order to engage the subfloor 4, and prevent rotation of the stem 14.

According to various embodiments, the stem 14 is displaced in relation to the main body 6 in the vertical direction by means of a jack arrangement, such as a tooth and pinion jack or a hydraulic jack. Thus, the elevating device 12 will be displaced in the vertical direction without turning in relation to the main body 6.

The decontamination apparatus 1 further comprises a nozzle device, generally designated 16, that comprises at least one nozzle 17. The nozzle device 16 is displaceable in the vertical direction between a retracted position and an extended position in relation to the main body 6. In figures 1 and 3 the nozzle device 16 is in the retracted position, and in figures 2 and 4 the nozzle device 16 is in the extended position. When the main body 6 is being attached to the flooring 3, the nozzle device 16 shall be in the retracted position in order to not obstruct proper connection of the decontamination apparatus 1 to the flooring 3. When the main body 6 is connected to the flooring 3 and the elevating device 12 is in the extended position, the nozzle device 16 may be displaced from the retracted position to the extended position, and when the nozzle device 16 is located in the extended position the at least one nozzle 17 is configured to be located in the space 5 between the flooring 3 and the subfloor 4. Said at least one nozzle 17 is configured to spray a decontamination liquid 19 into the space 5 between the flooring 3 and the subfloor 4.

When the floor 2 comprises a subsurface layer located between the flooring 3 and the subfloor 4, especially in situations when the subsurface layer is water/vapor non-permeable but also in situations when the subsurface layer is water/vapor permeable, one may need to secure that the decontamination liquid 19 reaches the portion of the space 5 located below the subsurface layer and/or reaches the portion of the space 5 located above the subsurface layer, depending on the location of the microbial fouling.

According to various embodiments, the distribution/spraying of the decontamination liquid 19 into the space 5 is performed in two rounds. One round entails spraying above the subsurface layer, and the other round entails spraying underneath the subsurface layer. In order to gain access to the portion of the space 5 located below the subsurface layer, an opening overlapping the opening 7 in the flooring 3 is provided in the subsurface layer. Thereafter, suitable lifting means are provided between the subfloor 4 and the subsurface layer in order to be able to distribute/spray the decontamination liquid 19 into the portion of the space 5 located below the subsurface layer by means of the nozzle device 16. The decontamination apparatus 1 may be removed between the two rounds in order to lift the subsurface layer before the second round. Alternatively the nozzle device 16 is configured to be located at different heights having the decontamination apparatus 1 connected to the flooring 3, wherein the flooring 3 is in the elevated position and the subsurface layer lifted from the subfloor 4, i.e. the nozzle device 16 is in the first round located at a level above the subsurface layer and in the second round located at a level below the subsurface layer, respectively.

According to various embodiments, the distribution/spraying of the decontamination liquid 19 into the space 5 is performed in one single round. The decontamination apparatus 1 is connected to the flooring 3, the flooring 3 being in the elevated position and the subsurface layer being lifted from the subfloor 4, and the nozzle device 16 is configured to spray above the subsurface layer and below the subsurface layer simultaneously.

The lifting means configured to lift the subsurface layer from the subfloor 4, may be constituted by separate elements or be part of the decontamination apparatus 1.

According to various embodiments, the nozzle device 16 comprises a pipe 18 that is connected to the main body 6, wherein the pipe 18 is displaceable in the vertical direction in relation to the main body 6. When the nozzle device 16 is in the extended position, the nozzle device 16 extends through the opening 7 in the flooring 3, i.e. the pipe 18 of the nozzle device 16 extends through the opening 7 in the flooring 3. The pipe 18 may be configured to glide in relation to the main body 6, i.e. the nozzle device 16 is pushed down to reach the extended position and pulled up to reach the retracted position. According to alternative embodiments, the nozzle device 16 is dragged down by the elevating device 12 and/or pushed up by the elevating device 12 when the elevating device 12 is displaced between the retracted position and the extended position. According to various embodiments, the elevating device 12 and the nozzle device 16 are connected to each other and jointly displaceable in relation to the main body 6 between the retracted position and the extended position.

The at least one nozzle 17 of the nozzle device 16 is configured to be connected to a source 20 of pressurized decontamination liquid 19. According to various embodiments, the source 20 of pressurized decontamination liquid 19 is constituted by a pressure tank 21. The pressure in the tank 21 is preferably generated by pumping gas/air into the tank 21, for instance by means of a manual hand pump. Alternatively the pressure in the tank 21 may be accomplished by means of a propellant gas. According to various embodiments, the decontamination apparatus 1 further comprises a conduit 22 that is releasably connected to the nozzle device 16 and to the source 20 of pressurized decontamination liquid 19. According to various embodiments the conduit 22 extending between the nozzle device 16 and the source 20 of pressurized decontamination liquid 19 comprises a controllable valve 23. The controllable valve 23 is preferably connected to the tank 21. The decontamination liquid 19 is configured to render the microbial fouling that is present between the flooring 3 and the subfloor 4 innocuous.

The inventive method for rendering microbial fouling between the flooring 3 and the subfloor 4 innocuous without removing the flooring 3, is performed by using a decontamination apparatus 1 as described above. The method comprises the steps of:
- forming an opening 7 in the flooring 3 in connection with a portion of the flooring 3 that is subject to microbial fouling,
- connecting the main body 6 of the decontamination apparatus 1 to the flooring 3 at said opening 7,
- elevating a portion of the flooring 3 from the subfloor 4 and creating a space 5 therebetween by means of the elevating device 12 of the decontamination apparatus 1, and
- applying pressurized decontamination liquid 19 into the space 5 between the flooring 3 and the subfloor 4 by means of the nozzle device 16 of the decontamination apparatus 1, wherein the nozzle device 16 comprises at least one nozzle 17 that is located in said space 5 during application of the pressurized decontamination liquid 19. The microbial fouling is essentially instantaneous rendered innocuous by the decontamination liquid 19.

According to various embodiments, the method also comprises the step of lowering the portion of the flooring 3 into contact with the subfloor 4 after the application of the pressurized decontamination liquid 19, in order to distribute/spread out the applied decontamination liquid 19 further.

According to various embodiments, the method further comprises the step of drying the flooring 3 and the subfloor 4 by providing an airflow into the space 5 between the flooring 3 and the subfloor 4. The airflow, may be untreated ambient air or dry and/or hot air. The airflow may be provided into the space 5 by blowing the air into the space generated between the flooring 3 and the subfloor 4, and/or by sucking moist air from the space 5 between the flooring 3 and the subfloor 4. The drying is preferably made according to conventional methods for drying water damaged floor. The lifting means configured to lift the subsurface layer from the subfloor 4, may also be used during the drying of the floor 2, in order to secure that the both the portion of the space 5 located below the subsurface layer and the portion of the space 5 located above the subsurface layer is properly dried. The airflow may be provided through the opening 7 into the space 5, or may be provided into the space 5 through other dedicated opening(s). When the decontamination process is finished, and if needed the floor 2 is dried, the flooring 3 is lowered to its original state resting on the subfloor 4.

When the decontamination process is finished, and if needed the floor 2 is dried, the opening 7 in the flooring 3 is closed by means of a suitable lid, made of a suitable flooring material. The lid is preferably made by the same material as the original flooring 3, or is constituted by the same portion of the flooring 3 that was initially removed. The opening 7 may be enlarged after the decontamination process and before the lid is attached, in order to remove any remaining marks in the flooring 3 such as screw holes and/or for facilitating attachment of the lid, and/or in order to remove damaged portions of the flooring 3.

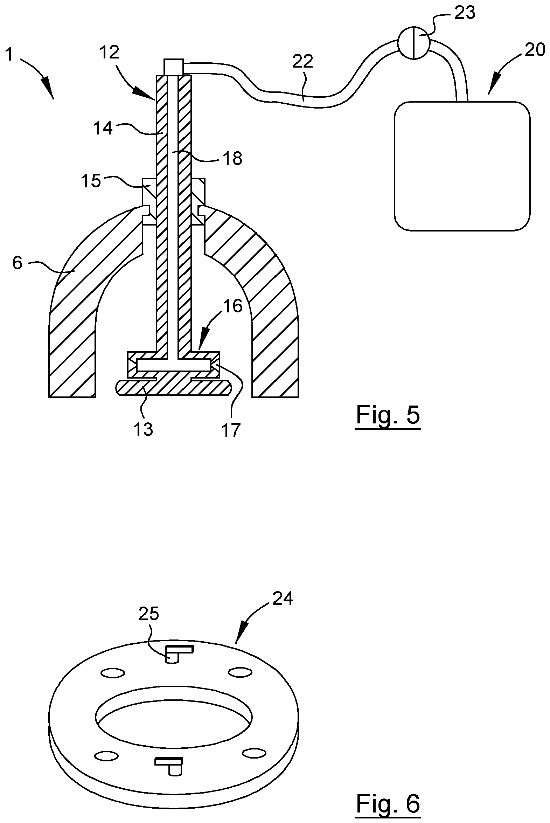

Reference is now made to figure 5 disclosing a decontamination apparatus 1 according to a third embodiment. The overall configuration and operation of the decontamination apparatus 1 is unchanged. According to this embodiment the nozzle device 16 and the elevation device 12 are connected to each other and jointly displaceable in relation to the main body 6. More precisely, the pipe 18 of the nozzle device 16 is incorporated into the stem 14 of the elevating device 12, and the at least one nozzle 17 of the nozzle device 16 is located adjacent the foot 13 of the elevating device 12. Thus, when the elevating device 12 is displaced between the retracted position and the extended position, the nozzle device 16 is automatically displaced between the retracted position and the extended position concurrently.

Reference is now made to figure 6 disclosing an adapter or attachment ring 24. The attachment ring 24 is configured to be connected to the flooring 3 at the opening 7. According to one example, the attachment ring 24 is connected to the top surface 8 of the flooring 3, for instance by means of a plurality of screws extending through the attachment ring 24. According to another example, the attachment ring 24 is connected to the flooring 3 by engaging the inner wall 10 of the opening 7. According to another example, the attachment ring 24 is connected to the flooring 3 by engaging the lower surface 11 of the flooring 3 adjacent the opening 7, for instance by hooks extended through the opening 7 and into the space 5. The main body 6 is connected to the attachment ring 24 in any suitable way, preferably by means of a quick-coupling arrangement. The lifting means configured to lift the subsurface layer from the subfloor 4, may be part of the attachment ring 24 or connected the attachment ring 24.

The attachment ring 24 may comprises engagement means, such as vertically extending pins 25. According to the disclosed embodiment the vertically extending pins 25 of the attachment ring 24 comprises horizontally extending members at the upper end of the pins 25. The main body 6 of the decontamination apparatus 1 comprises corresponding engagement means, such as bayonet coupling holes configured to cooperate with the vertically extending pins 25. Thus, the main body 6 is connected to the flooring 3 at the opening 7 via the attachment ring 24. Thereby, one and the same decontamination apparatus 1 may easily be used at multiple locations in the flooring 3, together with multiple attachment rings 24. Furthermore, the attachment ring 24 may also be used to connect a dryer apparatus to the flooring 3, after the decontamination apparatus 1 is removed.

According to various embodiments, the dryer is connected to the main body 6 of the decontamination apparatus 1.

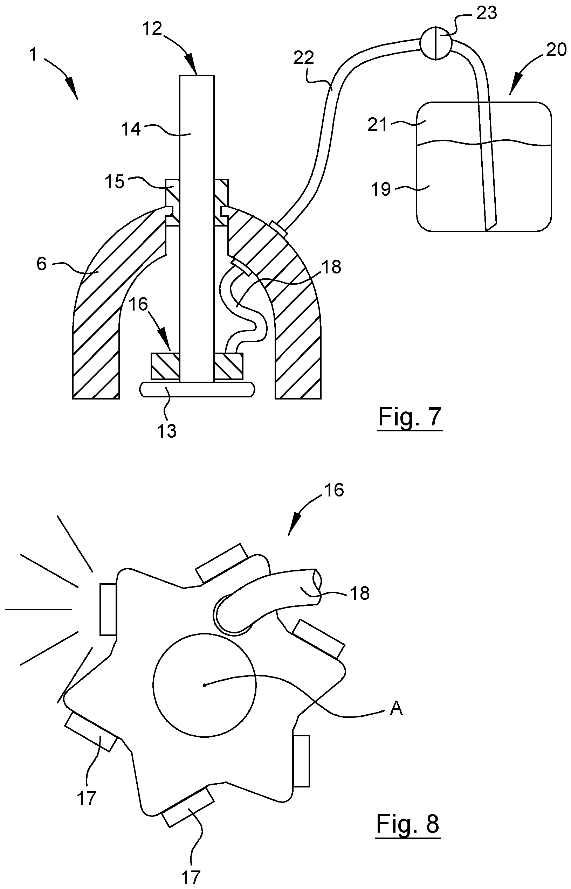

Reference is now made to figure 7 disclosing a decontamination apparatus 1 according to a fourth embodiment. The overall configuration and operation of the decontamination apparatus 1 is unchanged. According to this embodiment the nozzle device 16 and the elevation device 12 are connected to each other and jointly displaceable in relation to the main body 6. The nozzle device 16 is attached to the lower part of the stem 14 of the elevation device 12, and the pipe 18 is constituted by a flexible pipe connected to a wall-entrance located in the main body 6. The flexible pipe 18 admit vertical displacement of the nozzle device 16. According to various embodiments, the nozzle device 16 is rotatably arranged about the stem 14 of the elevation device 12, such that the stem 14 may be rotated in relation to the main body 6 in order to displace the elevation device 12 between the retracted position and the extended position without rotating the nozzle device 16 in relation to the main body 6. The conduit 22 is releasably connected to the outer end of the wall-entrance.

Reference is now made to figure 8 disclosing an example embodiment of a nozzle device 16 used together with the decontamination apparatus 1 disclosed in figure 7. The disclosed nozzle device 16 comprises six nozzles 17, wherein the nozzles 17 are equidistantly located about a vertical centre axis A, i.e. the stem 14. Thereby the spraying of the pressurized decontamination liquid 19 is performed in all direction around the stem 14 at the same time. According to various embodiments, the plurality of nozzles 17 are directed radially outwards. According to alternative embodiments, as disclosed in figure 8, the nozzles 17 are directed in tangential directions in relation to the vertical centre axis A. Thereby, when the decontamination liquid 19 is dispensed, the nozzle device 16 may automatically turn a little around the stem 14 and provide an even more optimal spraying/ distribution of the decontamination liquid 19 into the space 5.

According to the invention the nozzle device 16 comprises at least one nozzle 17. When one nozzle 17 is used, the decontamination apparatus 1 is preferably used when the opening 7 in the flooring 3 is located at the edge of the area effected by microbial fouling. When one or a few nozzle(s) 17 is used, the nozzle device 16 is preferably rotatable in order to direct the spraying in multiple directions in relation to the decontamination apparatus 1. According to various embodiments, when one or a few nozzle(s) 17 is used, the entire decontamination apparatus 1 may be rotated in order to direct the spraying in multiple directions. According to various embodiments, the nozzle device 16 comprises a plurality of nozzles 17, preferably at least three nozzles and most preferably at least six nozzles. When a plurality of nozzles 17 is used, they are preferably equidistantly located about a vertical centre axis A, i.e. the stem 14, in order to spray decontamination liquid 19 in all directions concurrently. By having more nozzles 17 the spray-pattern from each nozzle 17 may be configured to reach a longer distance from the nozzle 17. The spray-pattern from each nozzle 17 preferably partly overlap the spray-pattern from the adjacent nozzles 17. According to various embodiments, also a single nozzle 17 may be configured to spray in all directions in relation to the stem 14.

According to various embodiments, during the elevation of the flooring 3, the height of the space 5 at the opening 7 is usually in the range up to 5 centimetres and will not cause any mechanical damage to the flooring 3. Too low height of the space 5 entails inadequate distribution of the decontamination liquid 19, and too big height of the space 5 entails unnecessary load on the attachment arrangement between the decontamination apparatus 1 and the flooring 3 and unnecessary load on the flooring 3. Such elevation of the flooring 3 entails that the radius of the provided space 5 is about 75-150 centimetres, and the nozzle device 16 is preferably configured to spray the decontamination liquid 19 a distance of about 100-200 centimetres. The amount of decontamination liquid 19 added is preferably in the range 0,1-1 decilitre per square metre. Too little decontamination liquid 19 entails that there is a risk that all the microbial fouling is not made innocuous, and too much decontamination liquid 19 entails unnecessary soaking of the flooring 3 and subfloor 4.

According to various embodiments, the decontamination liquid 19 is constituted by a liquid product/compound that removes/kills microbial fouling such as mould and bacteria. According to various embodiments the decontamination liquid 19 is constituted by an alcohol compound, for instance comprising ethanol and water. The total amount of alcohol in the compound is preferably equal to or more than 50 % by volume, and up to 85 %. Most preferably, the total amount of alcohol in the compound is in the range 65-75 %. The alcohol compound based on ethanol is preferably denaturised in conventional way by addition of one or more other alcohols, such as propanol, methanol, etc., in the amount 5-20%, in order to prevent consumption.

### Feasible modifications of the Invention

The invention is not limited only to the embodiments described above and shown in the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims. Any subject matter falling outside the scope of the claims is provided for information purposes, and for placing the invention into a relevant context.

It shall also be pointed out that all information about/concerning terms such as above, under, upper, lower, etc., shall be interpreted/read having the equipment oriented according to the figures, having the drawings oriented such that the references can be properly read. Thus, such terms only indicate mutual relations in the shown embodiments, which relations may be changed if the inventive equipment is provided with another structure/design.

Throughout this specification and the claims which follows, unless the context requires otherwise, the word "comprise", and variations such as "comprises" or "comprising", will be understood to imply the inclusion of a stated integer or steps or group of integers or steps but not the exclusion of any other integer or step or group of integers or steps.

## Claims

1. Decontamination apparatus (1) for rendering microbial fouling between a flooring (3) and a subfloor (4) innocuous without removing the flooring (3),
**characterized in that** the decontamination apparatus (1) comprises:
- a main body (6) configured to be connected to the flooring (3) at an opening (7) in the flooring (3),
- an elevating device (12) that is connected to the main body (6) and is displaceable in the vertical direction between an extended position and a retracted position in relation to the main body (6), wherein the elevating device (12) is configured to engage the subfloor (4) when located in the extended position, in order to elevate a portion of the flooring (3) from the subfloor (4) and create a space (5) therebetween, and
- a nozzle device (16) that comprises at least one nozzle (17), wherein the nozzle device (16) is displaceable in the vertical direction between an extended position and a retracted position in relation to the main body (6), wherein said at least one nozzle (17) is configured to be located in said space (5) between the flooring (3) and the subfloor (4) when the nozzle device (16) is located in the extended position,
wherein said at least one nozzle (17) of the nozzle device (16) is configured to be connected to a source (20) of pressurized decontamination liquid (19).

2. The decontamination apparatus (1) according to claim 1, wherein the elevating device (12) is configured to extend through the opening (7) in the flooring (3) when located in the extended position.

3. The decontamination apparatus (1) according to claim 1 or 2, wherein said nozzle device (16) is configured to extend through the opening (7) in the flooring (3) when located in the extended position.

4. The decontamination apparatus (1) according to any preceding claim, wherein the elevating device (12) and the nozzle device (16) are connected to each other and jointly displaceable in relation to the main body (6).

5. The decontamination apparatus (1) according to any preceding claim, wherein the nozzle device (16) comprises at least three nozzles (17), preferably at least six nozzles (17).

6. The decontamination apparatus (1) according to claim 5, wherein the plurality of nozzles (17) are equidistantly located about a vertical centre axis (A).

7. The decontamination apparatus (1) according to any preceding claim, wherein the source (20) of pressurized decontamination liquid (19) is constituted by a pressure tank (21), and wherein a conduit (22) extends between the nozzle device (16) and said tank (21), wherein the conduit (22) comprises a controllable valve (23).

8. The decontamination apparatus (1) according to any preceding claim, wherein the flooring (3) is constituted by a wooden flooring.

9. Method for rendering microbial fouling between a flooring (3) and a subfloor (4) innocuous without removing the flooring (3), by using a decontamination apparatus (1), wherein the method comprises the steps of:
- forming an opening (7) in the flooring (3) in connection with a portion of the flooring (3) that is subject to microbial fouling,
- connecting a main body (6) of the decontamination apparatus (1) to the flooring (3) at said opening (7),
- elevating a portion of the flooring (3) from the subfloor (4) and creating a space (5) therebetween by means of an elevating device (12) of the decontamination apparatus (1), and
- applying pressurized decontamination liquid (19) into the space (5) between the flooring (3) and the subfloor (4) by means of a nozzle device (16) of the decontamination apparatus (1), wherein the nozzle device (16) comprises at least one nozzle (17) that is located in said space (5) during application of the pressurized decontamination liquid (19).

10. The method according to claim 9, wherein the method after the step of applying pressurized decontamination liquid (19), further comprises the step of:
- lowering the portion of the flooring (3) into contact with the subfloor (4) after the application of the pressurized decontamination liquid (19), in order to further spread out the applied decontamination liquid (19).

11. The method according to claim 9 or 10, wherein the method further comprises the step of:
- drying the flooring (3) and the subfloor (4) by providing an airflow into the space (5) between the flooring (3) and the subfloor (4).

12. The method according to any of claims 9-11, wherein the method also comprises the final step of:
- closing the opening (7) in the flooring (3).
